(19) Europäisches Patentamt
European Patent Office
Office européen des brevets

(11) Publication number: **0 031 965**
**A2**

(12) # EUROPEAN PATENT APPLICATION

(21) Application number: **80108230.6**

(22) Date of filing: **30.12.80**

(51) Int. Cl.[3]: **C 07 C 29/00**
C 07 C 29/17, C 11 B 9/00
C 07 C 35/37, C 07 C 49/753
C 07 D 309/12, C 07 F 7/02

(30) Priority: **04.01.80 CH 31/80**
**05.12.80 CH 9008/80**

(43) Date of publication of application:
**15.07.81 Bulletin 81/28**

(84) Designated Contracting States:
**CH DE FR GB LI NL**

(71) Applicant: **Roure Bertrand Dupont Société Anonyme**
**55, Voie des Bans**
**F-95102 Argenteuil(FR)**

(72) Inventor: **Bertrand, Marcel**
**8, avenue Guy de Maupassant**
**F-13008 Marseille(FR)**

(72) Inventor: **Pelerin, Gérard**
**20, rue Gillibert**
**F-13005 Marseille(FR)**

(72) Inventor: **Teisseire, Paul José**
**14, avenue Pierre Sémard**
**F-06332 Grasse(FR)**

(74) Representative: **Lederer, Franz, Dr. et al,**
**Patentanwälte Dr. Franz Lederer Reiner F. Meyer**
**Lucile-Grahn-Strasse 22**
**D-8000 München 80(DE)**

(54) **Process for the preparation of tricyclic compounds, intermediates useful in said process, novel tricyclic compounds so prepared and the use thereof as odoriferous agents.**

(57) A process for the preparation of tricyclic compounds is described in which novel intermediate compounds of the general formula

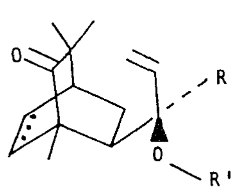

V

in which R represents hydrogen or alkyl having up to 5 carbons, the dotted line represents an optional carbon-carbon bond and OR' represents a leaving group is cyclised using an alkali metal in an organic solvent, followed, if desired, by hydrogenation to give compounds of the formulae

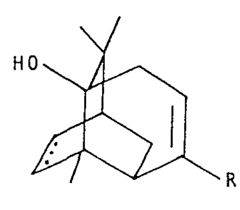

I and

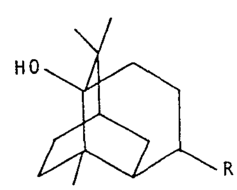

VI

Compounds of formula I and VI are useful as odoriferous agents having patchouli type odours and, with the exception of norpatchoulenol, are new.

EP 0 031 965 A2

30. Dez. 1980 0031965

Société Anonyme Roure Bertrand Dupont, 55, Voie des Bans, Argenteuil, France

Ref. 6600/31

# Process for the preparation of tricyclic compounds, intermediates useful in said process, novel tricyclic compounds so prepared and the use thereof as odoriferous agents.

The present invention relates to a process for the preparation of tricyclic compounds, as well as to intermediate compounds useful in this process. The tricyclic compounds so prepared are useful as odoriferous agents.

As will be particularly specified hereinafter in a more detailed manner, some of these tricyclic compounds are novel. These novel compounds constitute a particular aspect of the invention by virtue of their interesting olfactory properties.

The key reaction of the synthesis of the present invention is a cyclisation reaction.

Thus, the invention relates to a process for the preparation of tricyclic compounds, characterised in that a compound of the general formula

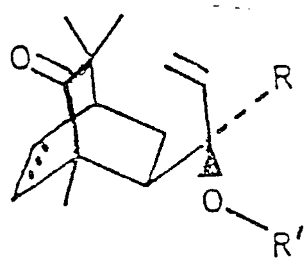

V

in which R represents a hydrogen atom or an alkyl group containing from 1 to 5 carbon atoms, the dotted line represents an optional carbon-carbon bond, and OR' represents a leaving group,

is cyclised by reaction with an alkali metal in an organic solvent to give a compound of the general formula

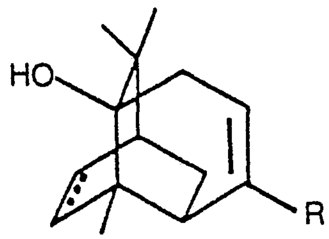

I

wherein R and the dotted line have the meanings given above,

and, if desired, the said compound of formula I is hydrogenated to yield a tricyclic compound of the general formula

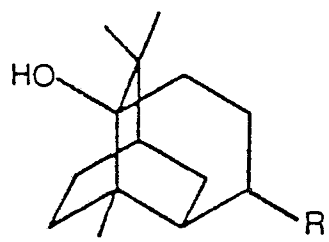

VI

wherein R has the meaning given above.

The radical R' may be any group which, together with the adjacent oxygen atom, forms an appropriate leaving group under the conditions of the cyclisation reaction. Examples of R' include: alkyl, alkoxyalkyl, pyranyl, tetrahydropyranyl and trialkylsilyl, wherein the alkyl and alkoxy groups contain from 1 to 5 carbon atoms. The alkyl group is preferably methyl or ethyl and the alkoxy group preferably methoxy or ethoxy. R' preferably represents a methoxymethyl, methyl, tetrahydropyranyl or trimethylsilyl group.

The cyclisation may be effected in any convenient organic solvent. The solvent used is normally a polar aprotic organic solvent such as tetrahydrofuran or dimethoxyethane.

The cyclisation reaction may conveniently be carried out at a temperature of up to the reflux temperature of the reaction mixture, preferably at a temperature of from 60 to 100°C. The reaction can be effected at the ambient

pressure and in the presence of any alkali metal, preferably lithium, sodium or potassium.

The novel tricyclic compounds according to the present invention have the general formula

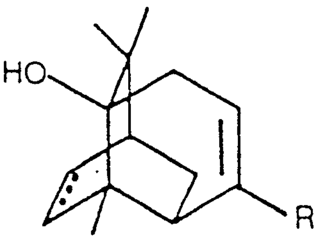

I

in which R represents a hydrogen atom or an alkyl group containing from 1 to 5 carbon atoms and the dotted line represents an optional carbon-carbon bond, with the proviso that where said optional carbon-carbon bond is absent, R is different from hydrogen.

The group R can be any straight or branched chain alkyl group having from 1 to 5 carbon atoms such as, for example, methyl, ethyl, n-propyl, isopropyl, n-butyl, isobutyl, t-butyl or n-pentyl. R preferably represents methyl.

Specific compounds of formula I which constitute preferred embodiments of the invention are compounds of the formulae:

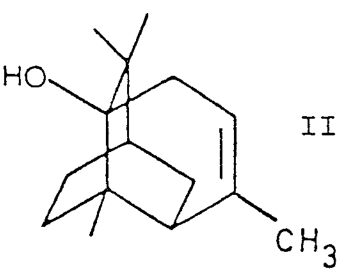

II

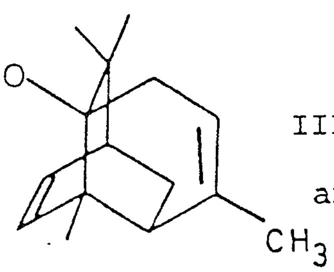

III

and

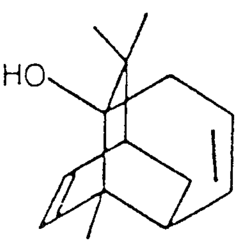

IV

The starting material of general formula V above, used in the process according to the present invention, may be prepared in any convenient manner. One such procedure, starting from 2,2,6-trimethyl-cyclohexadien-2,4-one which leads to compounds of general formula V, is set out below:

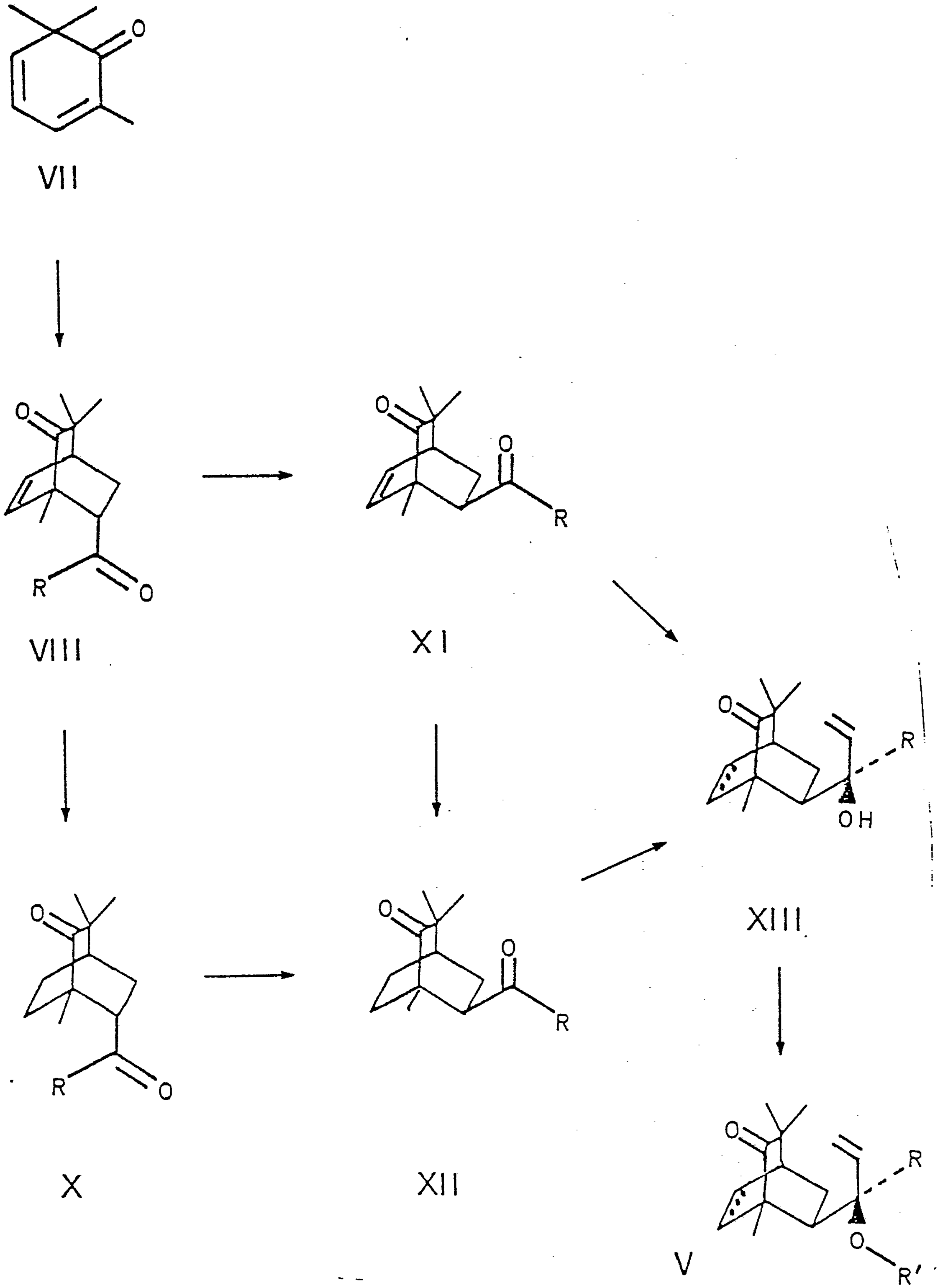

VII

VIII

XI

XIII

X

XII

V

2,2,6-Trimethyl-cyclohexadien-3,5-one of general formula VII may be converted into the ring-unsaturated diketone of general formula VIII by reaction with a compound of general formula

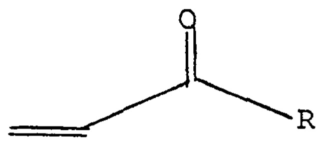

IX

wherein R has the meaning given above, namely hydrogen or an alkyl group having from 1 to 5 carbon atoms.

The dienone of general formula VII is conveniently used in the form of its dimer, since this results in a better yield. The dimer disassociates and yields the monomer in situ on refluxing in xylene. When acrolein is used as the compound of general formula IX (R = H), it should be added gradually in order to avoid polymerisation.

The diketone of formula VIII is then epimerised and, if desired, also hydrogenated depending on which substance of general formula V is desired. When not only the epimerisation but also the hydrogenation is carried out, the yield may vary depending on the order in which the two reactions are effected. Thus, where it is desired to produce compounds of general formula V, in which the dotted line is absent, the hydrogenation is preferably effected prior to the epimerisation. When the carbon-carbon bond represented by the dotted line is present, there will clearly not be any hydrogenation stage.

The hydrogenation reaction involving the conversion of compounds of formulae VIII and XI into compounds of formulae X and XII, respectively, can be carried out by any convenient method, for example by means of hydrogen in the presence of palladium-on-carbon. This hydrogenation may conveniently be carried out under pressure, at the ambient temperature and conveniently in an inert organic solvent, preferably a hydrocarbon solvent such as pentane.

The epimerisation reaction, in which the compounds of formula VIII and X are epimerised to the compounds of formulae XI and XII, respectively, can be carried out by any convenient method. The preferred method used will vary according to whether R represents hydrogen or an alkyl group, that is to say whether an aldehyde or a ketone side chain is being epimerised. In the case of a ketone, the reaction will be effected advantageously by means of sodium methylate in methanol as solvent. The reaction can be effected at any appropriate temperature, that is to say between room temperature and the reflux temperature of the solvent. Where an aldehyde is used as the starting material, the epimerisation may be effected by means of piperidinium acetate at the reflux temperature of the solvent.

The compounds of formulae XI and XII may be converted via the corresponding alcohol of formula XIII to the starting material for the process of the invention, namely the compounds of formula V. The alcohols of formula XIII may be prepared by the action of a vinyl-organometallic compound on a compound of formula XI or of formula XII. The preferred organometallic compounds are vinylmagnesium bromide or vinyl-lithium. The alcohol of formula XIII may then be converted into the corresponding ether of formula V by any convenient method. Where R' represents a methoxymethyl group, this can be carried out by reaction with triethylmethoxymethyl ammonium chloride in acetonitrile.

The hydrogenation of the compounds of formula I may be carried out by any convenient method, for example by means of hydrogen in the presence of a catalyst such as platinum or palladium, with or without a support such as carbon or Raney-nickel.

The compounds of general formula I possess, both in the solid state and in solution in organic solvents such

as those conventionally used in perfumery, powerful and intense odours of the general type of patchouli oil, but free from odours of the other odorant components of patchouli oil, in particular from the odours of the terpenes present in patchouli oil. The compound of formula II possesses a strong and warm odour of patchouli which has particular woody aspects and has an odour which is dry and has good tenacity. The compound of formula III has a strong and warm odour of patchouli with agreste features and which is gentle and soothing in nature. The compound of formula IV has a warm and strong odour of patchouli with spicy side notes. The compounds of general formula I may thus with advantage be used in many perfumes, as well as for perfuming various industrial products, such as solid and liquid detergents, synthetic washing agents, aerosols, and cosmetic products of all kinds. The amount in which the compounds of general formula I can be used in odorant compositions varies within wide limits. The compounds can thus be incorporated in products in amounts of from 0.5 to 20% by weight, preferably from 0.5 to 5% by weight.

Example 1

2 g of 2,6,6-trimethyl-cyclohexadien-3,5-one are placed in a 50 ml flask, provided with a side-tube, equipped with a reflux condenser and a dropping funnel. 15 ml of xylene are added and the mixture is refluxed for one hour. Then, 2 ml of acrolein are introduced portionwise over 90 minutes and the mixture is again refluxed for four hours. The solvent is then distilled off in vacuo and the product is distilled at 0.05 mm Hg. Boiling point 80-85°C. 2,6 g of 1,8,8-trimethyl-7-oxo-bicyclo-[2,2,2]-oct-5-ene-2-endo-carboxaldehyde (Yield 94%) are obtained.

Example 2

5 g of dimeric 2,2,6-trimethyl-cyclohexadien-3,5-one are placed in a 100 ml flask, equipped with a reflux condenser, 30 ml of xylene and 5.2 g of methyl vinyl ketone are added, then the mixture is refluxed for four hours. The solvent is then distilled off in vacuo and the product is rapidly distilled at 0.05 mm Hg. Boiling point 90-95°C. 7.27 g of 7-anti-acetyl-1,3,3-trimethylbicyclo-[2,2,2]oct-5-en-2-one (Yield 96%) are obtained.

Example 3

5 g of 1,8,8-trimethyl-7-oxo-bicyclo[2,2,2]oct-5-ene-2-endo-carboxaldehyde are placed in a 250 ml flask and 100 ml of pentane and 250 mg of 10% palladium-on-carbon are added. The mixture is then stirred for 12 hours at room temperature under a hydrogen atmosphere, filtered over Celite and the solvent is distilled off in vacuo. 1,5,5-Trimethyl-6-oxo-bicyclo[2,2,2]octane-2-endo-carboxaldehyde (Yield 95%) is obtained.

Example 4

The process of Example 3 is repeated using 7-anti-acetyl-1,3,3-trimethyl-bicyclo[2,2,2]oct-5-en-2-one as the starting material. There is thus obtained 6-oxo-acetyl-1,3,3-trimethyl-bicyclo[2,2,2]octan-2-one.

Example 5

2.05 g of piperidinium acetate are placed in a 50 ml flask, 25 ml of dry benzene and 2.20 g of 1,8,8-trimethyl-7-oxo-bicyclo[2,2,2]oct-5-ene-2-endo-carboxyldehyde are added and the mixture is heated for 4 hours at reflux. Then, the mixture is left to cool, poured into a separating funnel and 20 ml of water and 60 ml of diethyl ether are added. The ethereal phase is decanted, washed three times with 20 ml of water and dried over magnesium sulphate. There is thus obtained a mixture of the starting substance and of its epimer 1,5,5-trimethyl-6-oxo-bicyclo[2,2,2]octane-2-exo-carboxaldehyde. The two epimers are separated by distillation under reduced pressure.

Example 6

20.4 g of sodium hydride under oil is washed three times with 100 ml of dry petroleum ether under nitrogen with stirring. 600 ml of dry tetrahydrofuran and 66.3 g of 1,8,8-trimethyl-7-oxo-bicyclo[2,2,2]oct-5-ene-2-endo-carboxaldehyde are added, the temperature is raised to 40°C and subsequently the mixture is refluxed for 8 hours. After cooling, the mixture is poured into a well stirred phosphate buffer solution (680 g potassium biphosphate and 710 g disodium phosphate in 5 litres of water) under an inert atmosphere, extracted three times with 200 ml of petroleum ether and washed with 60 ml of the above buffer solution. After distillation off of the solvent, 68 g of a crude product is obtained (Yield 102%). Capillary vapour phase chromatography showed that the ratio of endo

to exo isomer was 20:80.

Example 7

720 mg of sodium methylate in solution in 50 ml of anhydrous methanol are introduced into a 100 ml flask, then 2.0 g of 7-anti-acetyl-1,3,3-trimethyl-bicyclo[2,2,2]-oct-5-en-2-one are added and the mixture is stirred for six hours at room temperature, refluxed for one hour and left to cool. The methanol is then distilled off in vacuo, the viscous residue is treated with 20 ml of water and 100 ml of diethyl ether and then neutralised with 10% hydrochloric acid. The organic phase is separated and the aqueous phase is again extracted with 30 ml of ether. The ethereal phases are combined and washed with 25 ml of a saturated solution of sodium chloride, then dried over magnesium sulphate. The solvent is evaporated and the epimeric 7-syn-acetyl-1,3,3-trimethyl-bicyclo[2,2,2]-oct-5-en-2-one is separated on a silica column (Eluant: pentane/ether 80:20) (Yield 0.86 g, 43%).

Example 8

The process of Example 7 is but repeated using 2 g of 6-exo-acetyl-1,3,3-trimethyl-bicyclo[2,2,2]octan-2-one as the starting material. There are thus obtained 1.4 g of 6-endo-acetyl-1,3,3-trimethyl-bicyclo[2,2,2]octan-2-one of melting point 51-52°C (Yield 70%).

Example 9

3.3 g of magnesium turnings under nitrogen are covered with 35 ml of anhydrous tetrahydrofuran in a 250 ml three-necked flask equipped with a reflux condenser, a dropping funnel and a stirrer. 1 ml of 1,2-dibromoethane is then added. After effervescence has ceased 13.4 g of vinyl magnesium bromide in the same weight of anhydrous tetrahydrofuran is slowly added so that the temperature

of the reaction medium remains between 35 and 45°C. 50 ml of anhydrous tetrahydrofuran are then added and the mixture refluxed for one hour followed by cooling to 0°C with continuous stirring. 19.4 g of 1,5,5-trimethyl-6-oxo-bicyclo[2,2,2]octane-2-exo carboxaldehyde diluted with the same weight of anhydrous tetrahydrofuran are then added with stirring and the temperature of the mixture is maintained between 0 and 10°C. The mixture is then allowed to warm up to the ambient temperature, poured into 200 g of ice and 60 g of ammonium chloride. The aqueous phase is extracted three times with 70 ml of diethyl ether. The combined organic phases are then washed three times with 50 ml of saturated aqueous sodium chloride and dried over magnesium sulfate. After purification on a silica column (Eluant: pentane-ether: 70-30) and recrystallisation from pentane, 9 g of white crystals of 6-endo-(1-hydroxyallyl)-1,3,3-trimethyl-bicyclo[2,2,2]octane-2-one are obtained (M.p. 93-94°C.)

## Example 10

1.6 g of 7-syn-acetyl-1,3,3-trimethyl-bicyclo[2,2,2]-octan-5-en-2-one are added to 8 ml of anhydrous tetrahydrofuran in a three necked 25 ml flask under a nitrogen atmosphere with stirring. The mixture is cooled to -20°C and 5 ml of a 2M solution of vinyl lithium in tetrahydrofuran is added. The temperature is then allowed to rise to 0°C and the reaction mixture is poured over 10 g of ice, decanted and the aqueous phase is extracted with 10 ml of diethyl ether, washed twice with 10 ml of saturated aqueous sodium chloride solution and dried over magnesium sulfate. After removal of the solvent and pufification on a silica column (Eluant: pentane-ether: 70-30) 1.2 g of 7-syn-(1-hydroxy-1-methylallyl)1,3,3-trimethyl-bicyclo[2,2,2]-oct-5-en-2-one is obtained (Yield: 85%).

Example 11

The procedure of Example 10 is repeated using 1.6 g of 6-endo-acetyl-1,3,3-trimethyl-bicyclo[2,2,2]octan-2-one as the starting material. There is thus obtained 1.3 g of 6-endo-(1-hydroxy-1-methylallyl)-1,3,3-trimethyl-bicyclo[2,2,2]octan-2-one (Yield: 90%).

Example 12

a) 130 ml of benzene, 15 g of triethylamine and 8.5 g of chloromethyl methyl ether are placed in a 250 ml flask. The mixture is then stirred for four hours with cooling. The product is filtered on glass frits, washed with 50 ml benzene and dried in a dessicator. 15.0 g of triethylmethoxymethyl ammonium chloride are obtained (Yield 78%), which are used as such in the following Examples.

b) 0.90 g of 6-endo(1-hydroxy-1-methyl-allyl)-1,3,3-trimethyl-bicyclo[2,2,2]-octan-2-one is placed in a 50 ml flask and 20 ml of acetonitrile and 1.1 g of triethylmethoxymethyl ammonium chloride are added. The mixture is refluxed for 24 hours, cooled, 12 ml of water are added and the mixture is extracted with ether, washed with a saturated solution of sodium chloride and dried over magnesium sulphate. After evaporation of the solvent and purification by passage through a silica column (Eluant: pentane-ether: 85-15), there is obtained 1.0 g of 6-endo-[1-(methoxymethoxy)-1-methyl-allyl]-1,3,3-trimethyl-bicyclo[2,2,2]octan-2-one (Yield: 95%).

Example 13

In a manner analogous to that described in Example 12, 7-syn-(1-hydroxy-1-methyl-allyl)-1,3,3-trimethyl-bicyclo[2,2,2]oct-5-en-2-one is converted into 7-syn-(1-methoxymethoxy-1-methyl-allyl)-1,3,3-trimethyl-bicyclo[2,2,2]oct-5-en-2-one. 1 g of product is obtained

(Yield: 95%).

Example 14

In a manner analogous to that described in Example 12, 6-endo-(1-hydroxy-allyl)-1,3,3-trimethyl-bicyclo-[2,2,2]octan-2-one is converted into 6-endo-(1-methoxy-methoxy-allyl)-1,3,3-trimethyl-bicyclo[2,2,2]octan-2-one. 1.0 g of product is obtained. (Yield: 95%).

Examle 15

180 mg of sodium pellets in suspension in toluene are placed in a 10 ml flask with a side-tube, equipped with a magnetic stirrer and a reflux condenser under an argon atmosphere. The toluene is evaporated off in vacuo. 4 ml of tetrahydrofuran, freshly distilled over naphthyl sodium in an atmosphere of argon, is slowly added, the mixture is refluxed, 500 mg of 6-endo-(1-methoxy-methoxy-1-methyl-allyl)-1,3,3-trimethyl-bicyclo[2,2,2]-octan-2-one in 1 ml of tetrahydrofuran are added and refluxing is continued for 1 hour. The reaction mixture is then cooled to room temperature and poured into 20 ml of diethyl ether. The remaining sodium is washed 3 times with 5 ml of anhydrous diethylether. The organic phase is washed neutral with a 5% solution of sodium chloride and dried over magnesium sulphate. After purification on a silica column (Eluant: pentane-ether: 85-15), there are obtained 190 mg of 4a,5,6,7,8,8a-hexahydro-4a,8a,9,9-tetramethyl-1,6-methanonaphthalen-1(2H)-ol.

Example 16

55 mg of lithium pellets in 4 ml of freshly distilled tetrahydrofuran are refluxed under an argon atmosphere and 757 mg of 6-endo-(1-methyl-1-tetrahydropyranyloxy-allyl)-1,3,3-trimethyl-bicyclo[2,2,2]octan-2-one in 1 ml of tetrahydrofuran. After refluxing for 4 hours the mixture

is cooled to room temperature and worked up as set forth in Example 15. After purification on a silica column (Eluant: pentane-ether: 85-15) there was isolated 83 mg of 4a,5,6,7,8,8a-hexahydro-4a,8a,9,9-tetramethyl-1,6-methanonaphthalen-1(2H)-ol.

Example 17

The process of Example 15 is repeated with 7-syn-(1-methoxymethoxy-1-methyl-allyl)-1,3,3-trimethyl-bicyclo[2,2,2]oct-5-en-2-one as the starting substance. There is thus obtained 4a,5,6,8a-tetrahydro-4,8a,9,9-tetramethyl-1,6-methanonaphthalen-1(2H)-ol.

Example 18

675 g of potassium pellets and 7.5 ml of tetrahydrofuran are refluxed under an argon atmosphere in the apparatus described in Example 15. 910 mg of 6-endo-(1-methoxymethoxyallyl)-1,3,3-trimethyl-bicyclo[2,2,2]octan-2-one in 1 ml of tetrahydrofuran are then added. After three hours refluxing the reaction mixture is cooled to room temperature and worked up as described above in Example 15. After purification on a silica column (Eluant: pentane-ether: 85-15) there are obtained 214 mg of norpatchoulenol.

Example 19

80 g of 4a,5,6,7,8,8a-hexahydro-4,8a,9,9-tetramethyl-1,6-methanonaphthalen-1(2H)-ol in 2 ml of anhydrous methanol are shaken for 12 hours under an atmosphere of hydrogen in the presence of 5 mg of 10% palladium-on-carbon. After filtration and evaporation of the solvent, there is obtained 0.66 mg of patchoulol, of which the spectra I.R., N.M.R. and Rf are identical to those obtained with a sample of natural patchoulol.

Examples 20 and 21 describe two odorant compositions

according to the invention containing a compound of general formula I.

Example 20

| Concentrate No 1 | Parts by weight |
|---|---|
| Oil of bergamotte peel extra | 30 |
| Oil of lemon peel | 20 |
| Ylang ylang Nossi-Bé oil | 50 |
| Phenylethyl alcohol | 266 |
| Hydroxycitronellal | 100 |
| Cyclamen aldehyde, 10% in ethyl phthalate (E.P.) | 10 |
| Aldehyde C.10 100%, 10% in E.P. | 5 |
| Aldehyde C.11 100%, 10% in E.P. | 5 |
| Lauric aldehyde C.12, 50% in E.P. | 20 |
| Petitgrain lemon-tree oil | 10 |
| Benzyl acetate | 60 |
| Phenylacetic acid, 1% | 10 |
| Iso-eugenol | 20 |
| Linalool | 20 |
| Anatolian rose oil | 30 |
| Geraniol | 20 |
| Jasmin absolute | 140 |
| Indolene liquid, 50% in E.P. | 20 |
| Oakmoss absolute A | 60 |
| Tree moss absolute | 20 |
| Methylionone | 50 |
| Resinoid Florentine iris washed | 10 |
| Phenylethyl acetate | 10 |
| Vetiver oil rectified | 20 |
| Vetiveryl acetate | 100 |
| Caryophyllenyl acetate | 60 |
| Guaiacum wood oil | 60 |
| Musk ambrette | 40 |
| Musk ketone | 20 |
| Coumarin | 20 |
| Cyclohexadecanolide | 50 |

| | |
|---|---|
| Kephalis (mixture of 1-ethoxy-4-(ethoxyvinyl)-3,3,5,5-tetramethylcyclo-1-hexene and 4-(1-ethoxyvinyl)-3,3,5,5-tetramethylcyclohexanone obtained following Example 2 of the French Patent 1498736) | 10 |
| Sandalwood oil | 20 |
| Compound of formula I (except norpatchoulenol itself) | 14 |
| | 1,400 |

## Example 21

| Concentrate No 2 | Parts by weight |
|---|---|
| Ylang ylang Nossi-bé oil | 40 |
| Oil of bergamotte peel | 240 |
| Oil of lemon peel | 120 |
| Benzyl acetate | 100 |
| Hydroxycitronellal | 140 |
| African geranium oil | 60 |
| Petitgrain of Grasse oil | 40 |
| Oil of neroli of Grasse | 10 |
| Phenylethyl alcohol | 40 |
| Lauric aldehyde C.12 50% (10% in E.P.) | 20 |
| Aldehyde C.12 MNA 100% (10% in E.P.) | 20 |
| Linalool | 50 |
| Aldehyde C.11 100% (10% in E.P.) | 20 |
| May rose absolute | 40 |
| Cyclamen aldehyde | 10 |
| Moss of holm oak absolute | 60 |
| Musk ketone | 60 |
| Coumarin | 80 |
| Jasmin absolute | 60 |
| Hexylcinnamic aldehyde | 30 |
| Resinoid No. 1 Styrax (50% in E.P.) | 30 |
| Kephalis | 50 |
| East Indies sandalwood oil | 20 |
| Vetiver oil rectified | 20 |
| Gamma methylionone | 40 |

| | |
|---|---|
| Musk ambrette | 90 |
| Cyclohexadecanolide | 30 |
| Compound of formula I (except norpatchoulenol itself) | 80 |
| | 1,600 |

CLAIMS

1. A process for the preparation of tricyclic compounds, characterised in that a compound of the general formula

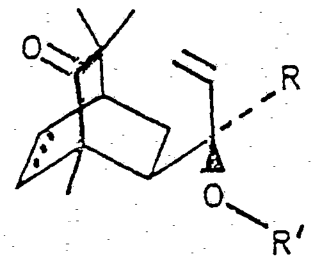

V

in which R represents a hydrogen atom or an alkyl group containing from 1 to 5 carbon atoms, the dotted line represents an optional carbon-carbon bond and OR' represents a leaving group,

is cyclised by reaction with an alkali metal in an organic solvent to give a compound of the general formula

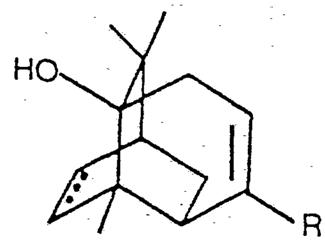

I

in which R and the dotted line have the meanings given above,

and, if desired, the said compound of formula I is hydrogenated to give a tricyclic compound of the general formula

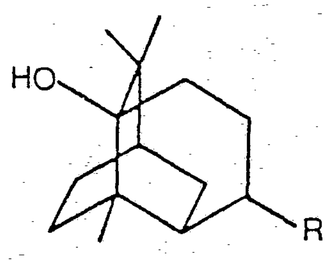

VI

wherein R has the meaning given above.

2. A process as claimed in claim 1, characterised in that R' represents an alkyl, alkoxyalkyl, pyranyl or silyl

group wherein the alkyl and alkoxy groups contain from 1 to 5 carbon atoms.

3. A process as claimed in claim 2, characterised in that R' represents a methoxymethyl, methyl or trimethylsilyl group.

4. A process as claimed in claim 1, characterised in that R' represent a tetrahydropyranyl group.

5. A process as claimed in any one of claims 1 to 4, characterised in that the cyclisation reaction is carried out in solution in a polar aprotic solvent.

6. A process as claimed in claim 5, characterised in that the solvent used is tetrahydrofuran or dimethoxyethane.

7. A process as claimed in any one of claims 1 to 6, characterised in that the temperature used for the cyclisation reaction is from 60 to 100°C.

8. A process as claimed in any one of claims 1 to 7, characterised in that the alkali metal used is lithium, sodium or potassium.

9. A process as claimed in any one of claims 1 to 8, characterised in that the starting material of general formula V is prepared by processes substantially as hereinbefore described.

10. Compounds of the general formulae

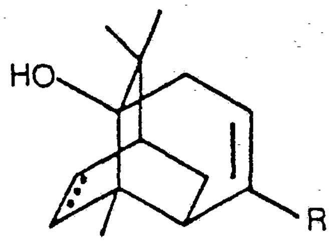 I or 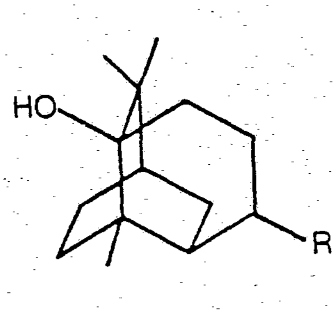 VI

wherein R and the dotted line have the meanings given in claim 1

whenever prepared by a process as claimed in any one of claims 1 to 9.

11. Compounds of the general formula

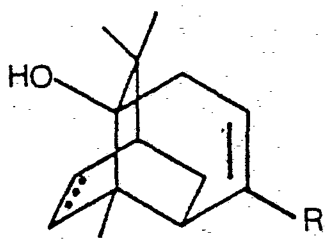

I

in which R represents a hydrogen atom or an alkyl group containing from 1 to 5 carbon atoms and the dotted line represents an optional carbon-carbon bond, with the proviso that where said carbon-carbon bond is absent R is different from hydrogen.

12. Compounds as claimed in claim 11, characterised in that R represents a methyl group.

13. The compound of formula

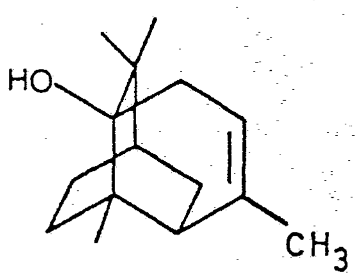

II

14. The compound of formula

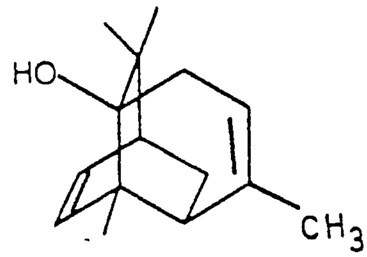

III

15. The compound of formula

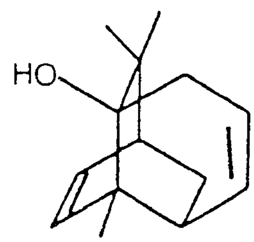

IV

16. Odoriferous compositions, characterised in that they contain a compound of general formula I as claimed in any one of claims 11 to 15.

17. Odoriferous compositions as claimed in claim 16, characterised in that they contain from 0.5 to 20% by weight of the said compound of general formula I.

18. Odoriferous compositions as claimed in claim 17, characterised in that they contain from 0.5 to 5% by weight of the said compound of general formula I.

19. The use of compounds of the general formula I as claimed in any one of claims 11 to 15 as odoriferous agents.

20. Compounds of the general formula I as claimed in any one of claims 11 to 15 as odoriferous agents.

21. Compounds of general formula

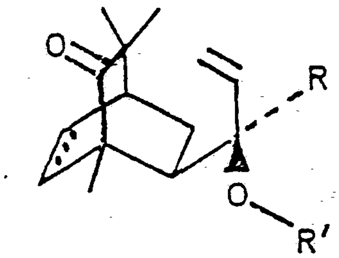

V

wherein R represents a hydrogen atom or an alkyl group containing from 1 to 5 carbon atoms, the dotted line represents an optional carbon-carbon bond and OR' represents a leaving group.

22. Compounds as claimed in claim 21, characterised in that R' represents an alkyl, alkoxyalkyl, pyranyl or silyl group wherein the alkyl and alkoxy groups contain from 1 to 5 carbon atoms.

23. Compounds as claimed in claim 21 or 22, characterised in that R' represents a methoxymethyl, methyl or trimethylsilyl group.

24. Compounds as claimed in claim 21, characterised in that R' represents a tetrahydropyranyl group.

***